# EUROPEAN PATENT SPECIFICATION

(11) **EP 3 220 888 B1**
(45) Date of publication and mention of the grant of the patent: **27.10.2021**
(21) Application number: 15801710.3
(22) Date of filing: 18.11.2015
(51) Int. Cl.: A61K 9/00, A61K 31/7048

(54) **EPRINOMECTIN PARENTERAL COMPOSITIONS**
PARENTERALE EPRINOMECTINZUSAMMENSETZUNGEN
COMPOSITIONS PARENTÉRALES D'ÉPRINOMECTINE

(30) Priority: 20.11.2014 EP 14306850
(43) Date of publication of application: 27.09.2017
(73) Proprietor: Ceva Santé Animale, 33500 Libourne (FR)
(72) Inventor: GUIMBERTEAU, Florence, 33450 Montussan (FR); FORGET, Patrick, 33700 Merignac (FR); COUSSANES, Evelyne, 33450 Izon (FR)
(74) Representative: Cabinet Becker et Associés
(86) International application number: PCT/EP2015/076947
(87) International publication number: WO 2016/079173

(56) References cited:
- WO-A2-2012/001083
- US-A1- 2004 198 704
- US-A1- 2007 042 013
- None

## Description

### FIELD OF THE INVENTION

This invention relates to compositions useful in the field of veterinary medicine and more particularly in relation to anti-parasitic formulations comprising eprinomectin, having a good stability over time, giving satisfactory therapeutic results and allowing for a zero milk withholding for lactating non human mammals.

### BACKGROUND OF THE INVENTION

Warm-blooded animals are subject to attack by parasites, and man has long sought to combat such parasites afflicting domestic companion animals, farmed livestock and exotic animals, to alleviate suffering and for commercial gain. Internal and external parasites, such as gastrointestinal worms and lice, are organisms that feed on a host animal's tissue, blood, and tissue fluids and can interfere with production. Internal parasites can cause appetite suppression, reduced feed digestibility and nutrient absorption, blood loss, and anemia, which in turn can lead to decreased weight gain and milk production, weakened immune system, and tissue and organ damage. External parasites can cause hair loss and scabbing, blood loss and anemia, and skin irritation, as well as acting as a disease vector. The effects of external parasites on production can include decreased weight gain and milk production, hide damage, and damage to facilities and fences from rubbing and scratching.

The macrocyclic lactones, such as for example, avermectins and milbemycins, are very potent antiparasitic agents at low dose and are useful against a broad spectrum of endoparasites and ectoparasites in mammals as well as having agricultural uses against various parasites found in and on crops and soils. They are active against many immature nematodes (including hypobiotic larvae) and arthropods.

The avermectins in commercial use are ivermectin, abamectin, doramectin, eprinomectin, and selamectin. Commercially available milbemycins are milbemycin oxime and moxidectin. The basic avermectin compounds are isolated from the fermentation broth of the soil micro-organism *Streptomyces avermitilis* and these compounds are described in US patent No. 4,310,519. Furthermore, derivatives of these basic avermectin compounds have been prepared by a variety of chemical means.

Specifically, eprinomectin, which is also named 4'-epi-acetylamino-4'-deoxy-avermectin B1, is a novel semi-synthetic derivative of the avermectin family characterized by the following structure:

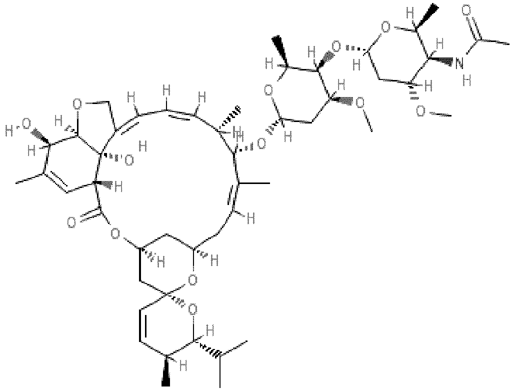

Eprinomectin is a mixture of two homologues, eprinomectin Bla (90%) and eprinomectin B1b (10%), the difference between them being the existence of a methylene group in C-25. These structures possess a wide activity spectrum against nematodes and anthropods and their effectiveness against both endo-and ectoparasites has led them to be called endectocides.

The pharmaceutical activity of these molecules increases the permeability of parasites muscle and nervous cells to chlorine ions, thus causing the parasite's paralysis and death. They bind to the glutamate-controlled chlorine channels of invertebrates' cells. They may also bind to other GABA-controlled chlorine channels. Since mammals do not possess this type of glutamate-controlled chlorine channel, these molecules provide a high degree of safety, even if used at high doses.

Eprinomectin-based products currently available in the market include formulations for external use such as pour-on formulations containing 0.5% w/v (g/100mL) eprinomectin solution which can be applied over the skin of the animal. Eprinomectin received a marketing authorization for topical pour-on administration, and is currently marketed by Merial under the trademark Eprinex^{®} or by Norbrook laboratories under the trademark Eprizero® for treating and/or preventing roundworms, lungworms, grubs, lice, mites, and horn flies in cattle. The Eprinex^{®} pour-on formulation currently comprises 5 mg eprinomectin, 0.10 mg butylhydroxytoluene, 0.06 mg Vitamin E and excipients per mL of formulation and Eprizero® pour-on comprises 5 mg eprinomectin, 0.10 mg butylhydroxytoluene, and excipients per mL of formulation.

The dose rate recommendation for these pour-on products is 1mL for each 10 kg of body weight corresponding to the eprinomectin recommended therapeutic dose: 0.5mg by kg of body weight.

Although pour-on methods have some advantages with regards to the applicator's safety, it is also largely affected by several factors which may reduce its efficacy due to the imprecision of its dosage. The uptake efficiency or adsorption of the drug is generally affected by the conditions of animal's skin like fur, presence of cuts, wounds, burns, as well as by the cleanness of the skin of animals (presence of dust, mud, etc...). Also, environmental factors (climatic conditions like rains, solar radiations, etc.) may affect the adsorption efficacy.

All these adverse conditions make it compulsory to more than double the necessary quantity of drug in order to combat the parasite. The requirement for this supplementary addition of eprinomectin has economic consequences regarding the cost per treated animal, may have a negative impact on the environment, and further can lead to potential side effects resulting from high drug dosage.

Eprinomectin has been suggested for use as injectable formulations. In fact, in addition to its endectocide activity, eprinomectin was shown not to be eliminated through milk fat in production animals. Studies concluded that only 0.1% of the applied drug was eliminated through the milk, that is to say, 50 times less when compared to other known macrocyclic lactones, such as, for example, ivermectin or moxidectin.

In that context, Longrange™ a ready-to-use sterile injectable preparation containing 5% w/v (g/100ml) of eprinomectin, is currently marketed by Merial. Each mL of Longrange™ contains 50 mg of eprinomectin in a co-solvent system of N-methyl-2-pyrrolidone (30% v/v) and triacetin (qs), along with 50 mg of poly-lactide-co-glycolic-acid 75:25 (PLGA), a polymer that allows a slow release of eprinomectin from the formulation, maintaining thereby a prolonged duration of product effectiveness. Butylated hydroxytoluene (0.2 mg/mL) acts as an antioxidant in the formulation. As described by Merial in the US marketing authorization (NADA #141-327), Longrange™ should be given only by subcutaneous injection in front of the shoulder at a recommended dosage level of 1 mg eprinomectin per kg body weight. Each mL of Longrange™ contains 50 mg of eprinomectin then 1mL is sufficient to treat 50 kg body weight. But, this injectable formulation animals intended for human consumption must not be slaughtered within 48 days of the last treatment. Longrange™ is not approved for use in female dairy cattle 20 months of age or older, including dry dairy cows. Use in these cattle may cause drug residues in milk and/or in calves born to these cows. A withdrawal period has not been established for pre-ruminating calves.

It has been described in US2007/042013 an injectable formulation comprising a bioactive agent, a subcutaneously volatile solvent, and a polymer. US2004/198704 discloses a composition comprising an antiparasitic agent and an antibacterial drug. Among a list of antiparasitic and antimicrobial agents, eprinomectin is cited. Eprinomectin is mentioned as highly potent when delivered topically. It has also been described in patent application WO 2012/001083 an injectable formulation comprising eprinomectin and a solvent system comprising polyethylene Glycol (PEG). It discloses said formulation increases drug bioavailability and residence time. The amount of eprinomectin in the composition is described as being comprised from 0.5 to 5%. The given example and the preferred formulation comprise 1 g of eprinomectin for a total volume of 100ml. This injectable eprinomectin formulation improves the bioavailability and the residence time of the parasiticide compared to an eprinomectin pour-on formulation. The injectable eprinomectin formulation described in patent application WO 2012/001083 allows a dosage level of eprinomectin around 0.5 mg per kg body weight compared to 1mg per kg body weight currently recommended for injectable eprinomectin formulation and therefore avoid any drug residues in milk and/or in calves born to these cows and then avoid any withdrawal period.

For parenteral injection, such as intramuscular or subcutaneous injections, as to reduce occasional discomfort or site reaction, it is recommended to inject the smallest possible volume of the formulation. One consequence is to inject once a high dose of the formulation (the active ingredient is thus concentrated in the formulation) which can create drug residues, scar tissue, and/or abscesses that can cause animal pain and suffering. For example, for bovine it is usual for intramuscular injections to inject in one site a maximum volume of 25 to 30 mL for adult cattle, from 10 to 20 mL for a young cattle and 5-10 mL for a calf. The corresponding maximum volumes for subcutaneous injections are 50 mL for an adult cattle, 20 mL for a young cattle and 10 mL for a calf. It is however recommended to divide volumes greater than 10 mL into two or more injection sites.

There is thus a need to have eprinomectin injectable formulations to be injected once in only one site but still stable over time, giving rise to satisfactory therapeutic results and that allow for a zero milk withholding for lactating dairy cows. Minor differences in formulations can have major impacts on the efficacy profile. A selection process was thus set up as to identify a vehicle for eprinomectin that would ensure maintenance of eprinomectin inherent positive qualities. In that context, dimethylsulfoxide has been identified to be a solvent and therefore a vehicle wherein eprinomectin maintains inherent positive qualities associated with an optimized administration (dissolution, syringeability ...).

### SUMMARY OF THE INVENTION

It is an object of the invention to provide a parenteral composition, comprising a therapeutically effective amount of eprinomectin and a vehicle comprising dimethylsulfoxide and optionally other excipients.

The composition of the invention allows controlling a broad range of endoparasites and ectoparasites in non-human mammals. Moreover, the parental composition presents an optimized syringeability.

This composition is particularly useful for treating and/or preventing infections caused by endoparasites and/or ectoparasites in non human mammals. The compositions of the invention are more specifically veterinary compositions. Compositions according to the present invention are particularly effective for controlling digestive and pulmonary strongyles.

A still further object of the invention is to provide a composition suitable for injection, comprising preferably from 1% to 10% (inclusive) of eprinomectin, and dimethylsulfoxide as solvent; the percentage corresponds to the weight of the ingredient expressed by gram for 100 ml of the composition (% w/v).

An essential object of the invention is to provide a veterinary composition comprising a therapeutically effective amount of eprinomectin and dimethylsulfoxide, for parenteral administration to non-human mammals, more particularly bovines, more specifically dairy cows.

The invention also provides a method for treating internal parasite infestations in a non-human mammal, comprising administering to said mammal an effective amount of the aforementioned composition.

The invention further provides a kit comprising a composition as disclosed herein and a package leaflet or user instructions including information that said composition is to be used for treating and/or preventing digestive and/or pulmonary strongyles in a non human mammal, preferably in a bovine or an ovine.

### DETAILED DESCRIPTION

An object of the invention is to provide a veterinary composition suitable for injection, comprising a therapeutically effective amount of eprinomectin and dimethylsulfoxide.

The present invention also relates to a veterinary composition suitable for injection, comprising a therapeutically effective amount of eprinomectin in a vehicle system comprising dimethylsulfoxide, and optionally other excipients.

It has now been surprisingly found that while eprinomectin is poorly lipophilic compared to other macrocyclic compounds (*i.e.*, moxidectin, doramectin, or ivermectin), injectable eprinomectin-based formulations may be prepared with an effective amount of dimethylsulfoxide solvent, and these formulations have improved properties in terms of residence time and consequently a higher lasting effect/duration of action when compared to other solvents. Further, the compositions according to the present invention may comprise a concentration of eprinomectin that allows administering en effective amount of eprinomectin in one injection site to non human mammals, preventing thereby animal pain and suffering. This allows good therapy compliance. Moreover, the compositions according to the invention show a satisfactory bioavailability, improved solubilizing properties of eprinomectin prior to administration, as well as a faster delivery of eprinomectin to the target sites. Furthermore, eprinomectin is known to be photosensitive and caution must be made when formulating said active ingredient. It has been found surprisingly that eprinomectin formulation with dimethylsulfoxide renders eprinomectin less sensitive to environmental factors such as light.

In the present description, the percentage corresponds to the weight of the compound or ingredient expressed by gram for 100 ml of the composition or formulation (% w/v), unless otherwise specified.

The formulation of the present invention preferably comprises from 1 to 10% w/v, more preferably from 2 % to 5%, and most preferably 2%, of eprinomectin. The eprinomectin would typically be completely dissolved in the formulation. A formulation of the invention may comprise from 1 to 70% w/v, preferably from 2% to 40% w/v, most preferably from 2.5% to 30 % w/v, most preferably 3%, of dimethylsulfoxide (DMSO).

The formulations of the invention may contain other inert ingredients such as antioxidants, preservatives, or pH stabilizers. These compounds are well known to one or ordinary skill in the formulation art.

Antioxidant such as an alpha tocopherol, ascorbic acid, ascrobyl palmitate, fumaric acid, malic acid, sodium ascorbate, sodium metabisulfate, n-propyl gallate, BHA (butylated hydroxyanisole), BHT (butylated hydroxytoluene), monothioglycerol and the like, may be added to the present formulation. In certain embodiments, the antioxidants are generally added to the formulation in amounts of from about 0.01 to about 2.0% w/v, with about 0.04 to about 1.0% (e.g.: 0.05%) being especially preferred.

In some embodiments, preservatives, such as the parabens (methylparaben and/or propylparaben), are suitably used in the formulation in amounts ranging from about 0.01 to about 2.0%, with about 0.05 to about 1.0% w/v being especially preferred. Other preservatives include benzalkonium chloride, benzethonium chloride, benzoic acid, benzyl alcohol, bronopol, butylparaben, cetrimide, chlorhexidine, chlorobutanol, chlorocresol, cresol, ethylparaben, imidurea, methylparaben, phenol, phenoxyethanol, phenylethyl alcohol, phenylmercuric acetate, phenylmercuric borate, phenylmercuric nitrate, potassium sorbate, sodium benzoate, sodium propionate, sorbic acid, thimerosal, and the like. Preferred ranges for these compounds include from about 0.01 to about 5 % w/v.

In a preferred embodiment, the composition according to the present invention further comprises at least another non-aqueous, polar or moderately polar, solvent. Said solvent is more particularly selected in the group consisting of glycerol formal, propylene glycol, N-methyl pyrrolidone, ethanol, propylene glycol mono- or di-ester, and a mixture thereof. According to a specific embodiment, in addition to dimethylsulfoxide (DMSO), the other solvent is glycerolformal (also known as 1,2-(methylidene)glycerol or 1,3-Dioxolane-4-methanol or 1,3-Dioxan-5-ol). The amount of glycerolformal, used as a solvent, in the composition of the invention may vary in a wide range; said quantity is preferably a quantity sufficient to attain the desired volume (Qsp).

Further a pH regulator may be used to adjust the pH in the range of about 5.5 to about 9.5. Examples of pH regulators for use according to the present invention include citric acid, acetate, phosphoric acid, ascorbic acid, gluconic acid, succinic acid, tartaric acid, lactic acid, and the like, their salts thereof, and mixtures thereof.

In a preferred embodiment, the present invention relates to a veterinary injectable composition comprising a therapeutically effective amount of eprinomectin, dimethylsulfoxide, glycerol formal and butylated hydroxytoluene. This is particularly useful for treating infections caused by endoparasites and/or ectoparasites in non-human mammals.

By "parenteral composition", "parenteral formulation", "injectable formulation" or "injectable composition" or "composition suitable for injection" is meant a formulation or composition that can be drawn into a syringe and injected subcutaneously, intravenously, intraperitoneally, or intra-muscularly into a non human mammals without causing adverse effects. According to a preferred embodiment of the present invention, the composition is administered to the non-human mammals by intra-muscular or subcutaneous injections.

As used herein, "about" or "around" will be understood by a person of ordinary skill in the art and will vary to some extent on the context in which it is used. If there are uses of the term which are not clear to persons of ordinary skill in the art given the context in which it is used, "about" or "around" will mean up to plus or minus 10% of the particular term.

In the formula according to the present invention, the eprinomectin in association with dimethylsulfoxide has improved stability and provides a satisfactory effect of the drug.

The veterinary eprinomectin composition for parenteral administration to non human mammals according to the present invention, can deliver eprinomectin at about 0.5 mg/kg body weight in order to be used at any stage of lactation and to keep residues very low. This property allows for zero milk withholding for lactating dairy cows.

Formulations of the present invention may be sterile and/or non-sterile injectable formulations. For instance, the formulation may be an aqueous or oleaginous suspension. The suspensions may be formulated according to techniques known in the art using suitable dispersing or wetting agents and suspending agents. The injectable formulation may also be a sterile injectable solution or suspension in a non-toxic parenterally acceptable diluent or solvent. Suitable diluents and solvents for injectable formulations include 1,3-butanediol, water, Ringer's solution and isotonic sodium chloride solution. Sterile, fixed oils are conventionally employed as a solvent or suspending medium. Suitable fixed oils include, but are not limited to, synthetic mono-or di-glycerides; fatty acids, such as oleic acid and its glyceride derivatives; and natural pharmaceutically-acceptable oils, such as olive oil, castor oil, and polyoxyethylated derivatives thereof (Sigma Chemical Co.; Fisher Scientific).

In addition, the formulation of the invention may further include one or more additional anti-parasitic agents, such as without any limitations, salicylanilides compounds, another additional avermectin and/or mylbemicins. In a particular embodiment, the formulation comprises at least two avermectins, more specifically a combination of eprinomectin and another avermectin. Among other avermectins, one can cite avermectins in commercial use, such as ivermectin, abamectin, doramectin, and selamectin.

Furthermore, the injectable formulation may further include any of the following many other ingredients in a pharmaceutically acceptable amount such as, for example, one or more crystallization inhibitors, colloids, adhesives, thickeners, thixotropic agents, penetrating agents, stabilizers, antioxidants, solubilizing agents, fluidizing agents, complexing agents, vitamins, minerals, preservatives, buffering agents, antiseptic agents or a combination thereof. More generally, the active ingredients may be combined with any solid or liquid additives corresponding to the usual techniques of formulation development. Examples of antioxidants include without limitation, sulfate compounds, L-cysteine, thiodipropionic acid, thiolactic acid, monothioglycerol, propyl galate sodium metabisulfite, sodium formaldehyde, sulfoxylate acetate, butylated hydroxyanisole (BHA) and butylated hydroxytoluene (BHT), vitamin E, ascorbic acid (vitamin C), vitamin B12, or a combination of these agents. Illustrative thickening agents include methylcellulose, hydroxyethyl cellulose, sodium carboxymethyl cellulose, hydroxypropyl cellulose, polyvinylpyrrolidone, and mixtures thereof. Illustrative complexing agents include EDTA and salts thereof, phosphate, nitrate, acetate, citrate, and mixtures thereof. Illustrative antiseptics include methyl p-oxybenzoate, propyl p-oxybenzoate, PHB ester, chlorobutanol, benzyl alcohol, butanol, butane-1,3-diol, chlorohexidin salts, benzoic acid and its salts, sorbic acid, and mixtures thereof.

The formulations of the present invention can be administered to non human mammals, especially mammals, including, but not limited to ruminant mammals, *e*.*g*., bovines or ovines, more generally cows, sheeps, goats, buffaloes, oxen, bulls, horses, pigs, cats, dogs, hamsters, mice, rats, monkeys, rabbits, deers, llamas, and yaks, and more particularly dairy and/or breeding cattle, such as cows and goats, more specifically dairy cows or dairy goats. In a preferred embodiment, the non human mammals are bovines. In most preferred embodiments, the non human mammals are milking cows or lactating cows or dairy cows.

The present invention further relates to a veterinary composition comprising a therapeutically effective amount of eprinomectin, and dimethylsulfoxide (as vehicle), optionally with other excipients, for parenteral administration to bovines, more specifically for dairy cows, in such a manner that less residues of eprinomectin, are found in the milk or in the meat and offal of cows as compared to pour on applications.

Actually, a surprising feature of the invention is that after administration of the formulation according to the present invention, the cows continue milking without delay with less residues of eprinomectin, and optionally any other macrocyclic lactone, in milk.

The present invention also relates to a veterinary injectable composition comprising a therapeutically effective amount of eprinomectin and dimethylsulfoxide as vehicle, optionally with other excipients, for controlling endoparasites and ectoparasites in non human mammals.

Such formulations are thus particularly useful for treating infections caused by endoparasites and/or ectoparasites in non human mammals, particularly in dairy cattle. Thus the formulation may be given to a healthy animal for the purpose of preventing a parasitic infection. The formulation may be given to an animal that is already infected, such as an animal that has suffered weight loss and diminution of milk production and quality (less protein and fat) due to the infection.

In various embodiments, the compositions used in the methods of the invention may further comprise a second pharmaceutically active compound. Representative classes of second pharmaceutically active compounds include, but are not limited to, other antibacterials, antifungals, antiparasitics, antivirals, and antiinflammatories.

Compositions and methods according to the present invention are particularly effective for controlling digestive and pulmonary strongyles and ectoparasites.

In a further aspect, the present invention relates to a method of treating infections caused by endoparasites and/or ectoparasites, which comprises administering to non-human mammals, preferably by injection, an effective amount of an anti-parasitic formulation as described herein.

According to this aspect, mammals which may be treated include, but are not limited to ruminant mammals, *e*.*g*., bovines or ovines, more generally cows, sheep, goats, buffaloes, oxen, bulls, horses, pigs, cats, dogs, hamsters, mice, rats, monkeys, rabbits, deers, llamas, and yaks, and more particularly dairy and/or breeding cattle. In most preferred embodiments the non human mammal is a milking cow or goat.

Within the context of the invention, the term treatment includes, particularly, the preventive treatment of non-human animals against an infection. The preventive treatment of a non-human animal against a disease designates a treatment made before the non-human animal has been exposed to or in contact with the causative agent of the disease (e.g., a pathogen, virus, protozoan, cell, etc.), or after said exposure/contact but before development of the symptoms or at an early stage of development of the disease. Also, the term preventive treatment, in relation to a population of non-human animals, designates the treatment of all members of the population even after the disease has been detected in certain members, to limit or avoid spreading of and contamination to the others.

In a particular embodiment, the term treatment designates the protection of a non-human animal against an infection, e.g., against the effect of an exposure to the causative agent, more specifically endoparasites and/or ectoparasites, or against the development of the disease in exposed non-human animals. The invention is particularly suited to protect non-human animals against an infectious disease such as a endoparasites or ectoparasites disease.

The term treatment also includes an increase in the welfare of the treated non-human animals, for example in increasing the production of meat, milk, wool, etc.

The term treatment also includes the alleviation of the symptoms, as well as a delay, reduction or cure of an existing infection.

In another embodiment for treatment against ectoparasites, the ectoparasite may be one or more insect or arachnid including those of the genera *Ctenocephalides, Rhipicephalus, Dermacentor, Ixodes, Ambylomma, Haemaphysalis, Hyalomma, Sarcoptes, Psoroptes, Otodectes, Chorioptes, Hypoderma, Damalinia, Linognathus, Haematopinus, Solenoptes, Trichodectes,* and *Felicola.*

In yet another embodiment for the treatment against ectoparasites, the ectoparasite is from the genera *Ctenocephalides, Rhipicephalus, Dermacentor, Ixodes* and/or *Boophilus.* The ectoparasites treated include but are not limited to fleas, ticks, mites, mosquitoes, flies, lice, blowfly and combinations thereof. Specific examples include but are not limited to cat and dog fleas (*Ctenocephalides felis, Ctenocephalides* sp. and the like), ticks (*boophilus* sp., *Ixodes* sp., *Dermacentor* sp., *Amblyoma* sp. and the like), and mites (*Demodex* sp., *Sarcoptes* sp., *Otodectes* sp. and the like), lice (*Trichodectes* sp., *Cheyletiella* sp., *Lignonathus* sp., and the like), mosquitoes (*Aedes* sp., *Culux* sp., *Anopheles* sp., and the like) and flies (*Hematobia* sp., *Musca* sp., *Stomoxys* sp., *Dematobia* sp., *Coclyomia* sp., and the like). In yet another embodiment for the treatment against ectoparasites, the ectoparasite is a flea and/or tick.

Additional examples of ectoparasites include but are not limited to the tick genus *Boophilus,* especially those of the species *microplus* (cattle tick), *decoloratus* and *anulatus*; myiases such as *Dermatobia hominis* (known as Berne in Brazil) and *Cochlyomia hominivorax* (greenbottle); sheep myiases such as *Lucilia sericata, Lucilia cuprina* (known as blowfly strike in Australia, New Zealand and South Africa), flies whose adult constitutes the parasite, such as *Haematobia irritans* (horn fly); lice such as *Linognathus vitulorum,* etc.; and mites such as *Sarcoptes scabici* and *Psoroptes ovis.* The above list is not exhaustive and other ectoparasites are well known in the art to be harmful to animals and humans. These include, for example migrating dipterous larvae.

The composition can also be used to treat against endoparasites such as those helminths selected from the group consisting of *Anaplocepheda, Ancylostoma, Anecator, Ascaris, Capillaria, Cooperia, Dipyllidinum, Dirofilaria, Echinococcus, Enterobius, Fasciola, Haemonchus, Oesophagostumum, Ostertagia, Toxocara, Strongyloides, Toxascaris, Trichinella, Trichuris,* and *Trichostrongylus.* Specifically bots, strongyles, ascarids and pinworms, roundworms like Wireworm*(Haemonchus placei),* Brown stomach worm *(Ostertagia spp.),* Nodular worm *(Oesophagostomum spp.),* Hookworm *(Bunostomum spp.),* Bankrupt worm *(Cooperia spp.),* Parafilaria bovicola(False *bruising), tapeworms like* milk tapeworm *(Moniezia spp.). Flukes, t*his group includes liver fluke species (*Fasciola* spp. - liver fluke and giant liver fluke) and conical fluke (*Calicophoron* - formerly *Paramphistomum*) the latter which occur in the gastrointestinal tract.

Further with or without the addition of additional pesticidal agents the composition of the invention can also be used to treat other pests which include but are not limited to pests:
➢ from the order Isopoda, for example *Oniscus asellus, Armadillidium vulgare* and *Porcellio scaber;*
➢ from the order Diplopoda, for example *Blaniulus guttulatus*;
➢ from the order Chilopoda, for example *Geophilus carpophagus* and *Scutigera* spp.;
➢ from the order Symphyla, for example *Scutigerella immaculata*;
➢ from the order Thysanura, for example *Lepisma saccharina*;
➢ from the order Collembola, for example *Onychiurus armatus*;
➢ from the order Orthoptera, for example *Acheta domesticus, Gryllotalpa* spp., *Locusta migratoria migratorioides, Melanoplus* spp. and *Schistocerca gregaria*;
➢ from the order Blattaria, for example *Blatta orientalis, Periplaneta americana, Leucophaea maderae* and *Blattella germanica*;
➢ from the order Dermaptera, for example *Forficula auricularia;*
➢ from the order Isoptera, for example *Reticulitermes* spp.;
➢ from the order Phthiraptera, for example *Pediculus humanus corporis, Haematopinus* spp., *Linognathus* spp., *Trichodectes* spp. and *Damalinia* spp.;
➢ from the order Thysanoptera, for example *Hercinothrips femoralis, Thrips tabaci, Thrips palmi* and *Frankliniella accidentalis*;
➢ from the order Heteroptera, for example *Eurygaster* spp., *Dysdercus intermedius, Piesma quadrata, Cimex lectularius, Rhodnius prolixus* and *Triatoma* spp.;
➢ from the order Homoptera, for example *Aleurodes brassicae, Bemisia tabaci, Trialeurodes vaporariorum, Aphis gossypii, Brevicoryne brassicae, Cryptomyzus ribis, Aphis fabae, Aphis pomi, Eriosoma lanigerum, Hyalopterus arundinis, Phylloxera vastatrix, Pemphigus* spp., *Macrosiphum avenae, Myzus* spp., *Phorodon humuli, Rhopalosiphum padi, Empoasca* spp., *Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus* spp. and *Psylla* spp.;
➢ from the order Lepidoptera, for example *Pectinophora gossypiella, Bupalus piniarius, Cheimatobia brumata, Lithocolletis blancardella, Hyponomeuta padella, Plutella xylostella, Malacosoma neustria, Euproctis chrysorrhoea, Lymantria* spp., *Bucculatrix thurberiella, Phyllocnistis citrella, Agrotis* spp., *Euxoa* spp., *Feltia* spp., *Earias insulana, Heliothis* spp., *Helicoverpa* spp., *Mamestra brassicae, Panolis flammea, Spodoptera* spp., *Trichoplusia ni, Carpocapsa pomonella, Pieris* spp., *Chilo* spp., *Pyrausta nubilalis, Ephestia kuehniella, Galleria mellonella, Tineola bisselliella, Tinea pellionella, Hofmannophila pseudospretella, Cacoecia podana, Capua reticulana, Choristoneurafumiferana, Clysia ambiguella, Homona magnanima, Tortrix viridana* and *Cnaphalocerus* spp.;
➢ from the order Coleoptera, for example *Anobiur punctatum, Rhizopertha dominica, Bruchidius obtectus, Acanthoscelides obtectus, Hylotrupes bajulus, Agelastica alni, Leptinotarsa decerlineata, Phaedon cochleariae, Diabrotica* spp., *Psylliodes chrysocephala, Epilachna varivestis, Atomaria* spp., *Oryzaephilus surinamensis, Anthonomus* spp., *Sitophilus* spp., *Otiorrhynchus sulcatus, Cosmopolites sordidus, Ceuthorrhynchus assimilis, Hypera postica, Dermestes* spp., *Trogoderma* spp., *Anthrenus* spp., *Attagenus* spp., *Lyctus* spp., *Meligethes aeneus, Ptinus* spp., *Niptus hololeucus, Gibbiur psylloides, Tribolium* spp., *Tenebrio molitor, Agriotes* spp., *Conoderus* spp., *Melolontha melolontha, Amphimallon solstitialis* and *Costelytra zealandica*;
➢ from the order Hymenoptera, for example *Diprion* spp., *Hoplocampa* spp., *Lasius* spp., *Monomorium pharaonis* and *Vespa* spp.;
➢ from the order Diptera, for example *Aedes* spp., *Anopheles* spp., *Culex* spp., *Drosophila melanogaster, Musca* spp., *Fannia* spp., *Calliphora erythrocephala, Lucilia* spp., *Chrysomyia* spp., *Cuterebra* spp., *Gastrophilus* spp., *Hyppobosca* spp., *Stomoxys* spp., *Oestrus* spp., *Hypoderma* spp., *Tabanus* spp., *Tannia* spp., *Bibio hortulanus, Oscinella frit, Phorbia* spp., *Pegomyia hyoscyami, Ceratitis capitata, Dacus oleae, Tipula paludosa, Hylemyia* spp. and *Liriomyza* spp.;
➢ from the order Siphonaptera, for example *Xenopsylla cheopis* and *Ceratophyllus spp.;*
➢ from the class of arachnids, for example *Scorpio maurus, Latrodectus mactans, Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp., Hemitarsonemus spp.* and *Brevipalpus spp.*

The compositions of the invention may be administered by injection(s) using techniques and/or devices known *per se* in the art. In this regard, injection can be performed with a device, such as syringe, a gun, a micro-needle injection device, a needle-free injection device, a pulse device, etc. In a preferred embodiment, injection is performed with a needle injector or a syringe.

According to a particular embodiment, the composition of the invention is administered to a non-human animal as a single administration to the animal. According to a specific embodiment, the composition of the invention is administered to a non-human animal as a single injection at one injection site of the animal.

According to another aspect of the invention, the composition is administered to said non-human mammals by injection, such as subcutaneous, intravenous, intraperitoneal, or intra-muscular injection, in particular via intra-muscular or subcutaneous injection, and preferably by subcutaneous injection.

Another object of the invention is a kit comprising a composition as disclosed herein and a package leaflet or user instructions including information that said composition is to be used for treating and/or preventing infections caused by endoparasites and/or ectoparasites in non human mammals, preferably bovines or ovines.

A further object of the invention is a kit comprising a composition as disclosed herein and a package leaflet or user instructions including information that said composition is to be used for treating and/or preventing digestive and/or pulmonary strongyles in a non human mammal, preferably in a bovine or an ovine.

Further aspects and advantages of the invention will be disclosed in the following illustrative experimental section.

### EXAMPLES

### Example 1 ― Preparation of injectable veterinary formulations of eprinomectin

Injectable veterinary eprinomectin composition was prepared by mixing the following components as showed in the Table 1 below (quantity expressed per 1 liter):

**Table 1:**

| | Formula 1 | Formula 2 | Formula 3 | Formula 4 | Formula 5 | Formula 6 |
|---|---|---|---|---|---|---|
| Eprinomectin | 10 g | 10 g | 20g | 20g | 20g | 100g |
| Dimethylsulfoxide | 30 g | 690g | 300g | 300g | 30g | 30g |
| BHT | - | - | 0.5g | 0.5g | 0.5g | 0.5g |
| Glycerol formal | - | - | - | 852g | 1156g | 1073g |
| Propylene glycol | 996g | 372g | 727g | - | - | - |

### Example 2- Dissolution rate

Dissolution rate is the time (in minutes) measured to solubilize totally (limpid solution) the eprinomectin in a specific carrier.

**Table 2:**

| | Time |
|---|---|
| Formula 5 | 50 |
| Formula 5 without DMSO | 90 |

Dimethylsulfoxide (DMSO) accelerates the dissolution of eprinomectin in a carrier comprising glycerol formal.

### Example 3- Physiochemical Stability Tests

Stability tests of formulas as described in example 1 are realized by following the two FDA guidelines VICH GL39 (Quality) dated November 2005 - International Cooperation on Harmonization of Technical Requirements for Registration of Veterinary Medical Products-"Test procedures and acceptance criteria for new veterinary drug substances and new medicinal products: Chemical substances" and VICH GL3(R) dated November 2007 - International Cooperation on Harmonization of Technical Requirements for Registration of Veterinary Medical Products- Guidance for Industry- "Stability testing of new veterinary drug substances and medicinal products (revision)"

Formula 1 to 4 and 6 are stable during 3 months at temperature between 4 and 40°C and Formula 5 is stable during 6 months at temperature between 4 and 40°C.

### Example 4- Stability Tests

Stability tests are conducted on Formula 5 as described by the US eprinomectin pharmacopeia (*USP31―NF26 Supplement: No. 1 Page 3628, Pharmacopeial Forum: Volume No. 33(1) Page 60)* in 100mL plastic vial at 2 temperatures (25°C, 30°C) and 2 Relative Humidity (RH) conditions (60% and 75%) during 18 months and at 40°C with 75% RH during 6 months.

**Table 3:**

| Plastic vial of 100 mL 25°C/60%RH | T0 | T3 months | T6 months | T12 months | T18 months |
|---|---|---|---|---|---|
| Relative density | 1.218 | 1.218 | 1.218 | 1.218 | 1.218 |
| Eprinomectin (mg/ml) | 19.9 | 20.0 | 19.7 | 19.6 | 19.8 |
| BHT (mg/ml) | 0.74 | 0.73 | 0.73 | 0.73 | 0.73 |
| Total impurities (%) | 0.6 | 0.6 | 0.6 | 0.6 | 0.6 |

**Table 4:**

| Plastic vial of 100 mL 30°C/75%RH | T0 | T3 months | T6 months | T12 months | T18 months |
|---|---|---|---|---|---|
| Relative density | 1.218 | 1.218 | 1.218 | 1.218 | 1.217 |
| Eprinomectin (mg/ml) | 19.9 | 19.9 | 19.7 | 19.7 | 19.7 |
| BHT (mg/ml) | 0.74 | 0.72 | 0.73 | 0.73 | 0.72 |
| Total impurities (%) | 0.6 | 0.6 | 0.6 | 0.6 | 0.6 |

**Table 5:**

| Plastic vial of 100 mL 40°C/75%RH | T0 | T3 months | T6 months |
|---|---|---|---|
| Relative density | 1.218 | 1.218 | 1.217 |
| Eprinomectin (mg/ml) | 19.9 | 19.9 | 19.6 |
| BHT (mg/ml) | 0.74 | 0.72 | 0.71 |
| Total impurities (%) | 0.6 | 0.6 | 0.6 |

Formula 5 is stable at least 18 months at temperature between 25 and 30°C with relative humidity between 60 and 75%. This formula 5 is still stable at least 6 months at high temperature and relative humidity (40°C/75% RH)

### Example 5- Syringeability tests

Here, syringeability is the Time measured in second(s) which is required to pass 10 ml of a solution in a glass syringe with a needle of 1.2 mm by applying a 1kg piston force.

**Table 6:**

| | Time |
|---|---|
| Formula 1 | 31 |
| Formula 5 | 12 |

### Example 6- Local tolerance at injection site

The local tolerance is assessed at the injection-site of eprinomectin composition (Formulas 4 and 5) administered via a unique sub-cutaneaous injection (1mL/100Kg) in 29 cows (adults and young bovines from 250 to 500Kg).

All injection sites were evaluated before and during 4 weeks after injection by means of circumferential and local temperature measurements, tumefaction, induration and subjective visible inflammation scores.

No adverse effect associated with formulas 4 and 5 injections was observed.

### Example 7- Milk eprinomectin milk Withdrawal Period determination

Formula 5 was administered by subcutaneous injection (1mL/100Kg) to 21 dairy cows (average weight between 200 to 500Kg) at a single injection point. The eprinomectin B1 levels (usual eprinomectin marker) were assayed on milk samples taken twice a day (morning and evening) during 10 days after injection.

All eprinomectin concentrations were below the Maximum Residue Level (MRL) fixed to 20mg/Kg, which justifies a zero hour milk withdrawal Period.

## Claims

1. A parenteral composition, comprising a therapeutically effective amount of an eprinomectin and a vehicle comprising dimethylsulfoxide, and optionally other excipients.

2. The composition according to claim 1, comprising from 1% to 10% w/v, preferably 2% w/v of eprinomectin.

3. The composition according to claim 1 or 2, comprising from 1 to 70% w/v, preferably from 2-40% w/v, more preferably 3 % w/v, of dimethylsulfoxide.

4. The composition according to any one of the preceding claims, wherein the composition further comprises at least one non-aqueous, polar or moderately polar, solvent.

5. The composition according to claim 4, wherein the non-aqueous, polar or moderately polar, solvent is selected in the group consisting of glycerolformal, propylene glycol, N-methyl pyrrolidone, ethanol, propylene glycol mono- or di-ester, and a mixture thereof, preferably is glycerolformal.

6. The composition according to any one of the preceding claims, further comprising one or more anti-parasitic agents, such as salicylanilides compounds, an additional avermectin and/or mylbemicins.

7. The composition according to any one of the preceding claims, further comprising one or more solid or liquid additives chosen from amongst protective, crystallization inhibitors, colloids, adhesives, thickeners, thixotropic agents, penetrating agents, stabilizers, antioxidants, solubilizing agents, fluidizing agents, complexing agents, vitamins, minerals, preservatives, buffering agents, antiseptics agents and combinations thereof.

8. The composition according to claim 7, wherein the antioxidant is selected in the group consisting of butylated hydroxyanisole, butylated hydroxytoluene, and a mixture thereof.

9. The composition according to any one of the preceding claims, for use in treating and/or preventing infections caused by endoparasites and/or ectoparasites in non human mammals.

10. The composition for use according to claim 9, for treating and/or preventing digestive and/or pulmonary strongyles in non human mammals.

11. The composition for use according to claim 9 or 10, wherein said non human mammals are bovines or ovines.

12. The composition for use according to claim 11, wherein said non-human mammals are dairy cattle or breeding cattle.

13. The composition for use according to any one of claims 9-12, wherein said composition is administered to said non human animal as a single administration.

14. The composition for use according to any one of the claims 9-13, wherein said composition is administered to said non human mammals via intra-muscular or subcutaneous injection, preferably via subcutaneous injection.

15. A kit comprising a composition according to any one of claims 1 to 8 and a package leaflet or user instructions including the information that said composition is to be used for treating and/or preventing digestive and/or pulmonary strongyles in a non human mammal, preferably a bovine or an ovine.

## Patentansprüche

1. Parenterale Zusammensetzung, umfassend eine therapeutisch wirksame Menge eines Eprinomectins und ein Vehikel umfassend Dimethylsulfoxid und gegebenenfalls andere Hilfsstoffe.

2. Zusammensetzung nach Anspruch 1, enthaltend 1 bis 10 % w/v, vorzugsweise 2 % w/v Eprinomectin.

3. Zusammensetzung nach Anspruch 1 oder 2, umfassend 1 bis 70 % w/v, vorzugsweise 2-40 % w/v, stärker bevorzugt 3 % w/v Dimethylsulfoxid.

4. Zusammensetzung nach einem der vorhergehenden Ansprüche, wobei die Zusammensetzung weiterhin mindestens ein nicht-wässriges, polares oder mäßig polares Lösungsmittel umfasst.

5. Zusammensetzung nach Anspruch 4, wobei das nichtwässrige, polare oder mäßig polare Lösungsmittel ausgewählt ist aus der Gruppe bestehend aus Glycerinformal, Propylenglycol, N-Methylpyrrolidon, Ethanol, Propylenglycolmono- oder -diester, und eine Mischung davon, vorzugsweise Glycerinformal ist.

6. Zusammensetzung nach einem der vorhergehenden Ansprüche, weiterhin enthaltend ein oder mehrere antiparasitäre Mittel, wie Salicylanilid-Verbindungen, ein zusätzliches Avermectin und/oder Milbemycine.

7. Zusammensetzung nach einem der vorhergehenden Ansprüche, weiterhin enthaltend ein oder mehrere feste oder flüssige Additive, ausgewählt aus Schutzmitteln, Kristallisationsinhibitoren, Kolloiden, Klebstoffen, Verdickungsmitteln, Thixotropiermitteln, Penetrationsmitteln, Stabilisatoren, Antioxidantien, Lösungsvermittlern, Fluidisierungsmitteln, Komplexbildnern, Vitaminen, Mineralstoffen, Konservierungsmitteln, Puffern, Antiseptika und Kombinationen davon.

8. Zusammensetzung nach Anspruch 7, wobei das Antioxidans ausgewählt ist aus der Gruppe bestehend aus butyliertem Hydroxyanisol, butyliertem Hydroxytoluol und einer Mischung davon.

9. Zusammensetzung nach einem der vorhergehenden Ansprüche zur Verwendung bei der Behandlung und/oder Vorbeugung von Infektionen, die durch Endoparasiten und/oder Ektoparasiten bei nicht menschlichen Säugetieren verursacht werden.

10. Zusammensetzung zur Verwendung nach Anspruch 9 zur Behandlung und/oder Vorbeugung von Verdauungs- und/oder Lungen-Strongyliden bei nicht-menschlichen Säugetieren.

11. Zusammensetzung zur Verwendung nach Anspruch 9 oder 10, wobei die nicht-menschlichen Säugetiere Rinder oder Schafe sind.

12. Zusammensetzung zur Verwendung nach Anspruch 11, wobei die nicht menschlichen Säugetiere Milchvieh oder Zuchtrinder sind.

13. Zusammensetzung zur Verwendung nach einem der Ansprüche 9-12, wobei die Zusammensetzung dem nicht-menschlichen Tier als einzelne Verabreichung verabreicht wird.

14. Zusammensetzung zur Verwendung nach einem der Ansprüche 9-13, wobei die Zusammensetzung den nicht-menschlichen Säugetieren durch intramuskuläre oder subkutane Injektion, vorzugsweise durch subkutane Injektion, verabreicht wird.

15. Kit, umfassend eine Zusammensetzung nach einem der Ansprüche 1 bis 8 und eine Packungsbeilage oder Gebrauchsanweisung, die die Information enthält, dass die Zusammensetzung zur Behandlung und/oder Vorbeugung von Verdauungs- und/oder Lungen-Strongyliden bei einem nicht menschlichen Säuger verwendet werden soll, vorzugsweise einem Rind oder einem Schaf.

## Revendications

1. Composition à usage parentéral, comprenant une quantité efficace du point de vue thérapeutique d'une éprinomectine et un véhicule comprenant du diméthylsulfoxyde, et éventuellement d'autres excipients.

2. Composition selon la revendication 1, comprenant de 1 % à 10 % en poids/volume, de préférence 2 % en poids/volume, d'éprinomectine.

3. Composition selon la revendication 1 ou 2, comprenant de 1 à 70 % en poids/volume, de préférence de 2 à 40 % en poids/volume, mieux encore 3 % en poids/volume, de diméthylsulfoxyde.

4. Composition selon l'une quelconque des revendications précédentes, laquelle composition comprend en outre au moins un solvant non aqueux polaire ou modérément polaire.

5. Composition selon la revendication 4, dans laquelle le solvant non aqueux polaire ou modérément polaire est choisi dans le groupe constitué par le glycérol-formal, le propylèneglycol, la N-méthylpyrrolidone, l'éthanol, un mono- ou di-ester de propylèneglycol, et leurs mélanges, de préférence est le glycérol-formal.

6. Composition selon l'une quelconque des revendications précédentes, comprenant en outre un ou plusieurs agents antiparasitaires, tels que des composés salicylanilides, une avermectine additionnelle et/ou des mylbémicines.

7. Composition selon l'une quelconque des revendications précédentes, comprenant en outre un ou plusieurs additifs solides ou liquides choisis parmi les protecteurs, les inhibiteurs de cristallisation, les colloïdes, les adhésifs, les épaississants, les agents thixotropes, les agents de pénétration, les stabilisants, les antioxydants, les agents solubilisants, les agents fluidisants, les agents complexants, les vitamines, les minéraux, les conservateurs, les agents tampons, les agents antiseptiques, et leurs combinaisons.

8. Composition selon la revendication 7, dans laquelle l'antioxydant est choisi dans le groupe constitué par l'hydroxyanisole butylé, l'hydroxytoluène butylé, et leurs mélanges.

9. Composition selon l'une quelconque des revendications précédentes, pour une utilisation dans le traitement et/ou la prévention d'infections provoquées par des endoparasites et/ou des ectoparasites chez des mammifères non humains.

10. Composition pour une utilisation selon la revendication 9, pour le traitement et/ou la prévention de strongles digestifs et/ou pulmonaires chez des mammifères non humains.

11. Composition pour une utilisation selon la revendication 9 ou 10, dans laquelle lesdits mammifères non humains sont des bovins ou des ovins.

12. Composition pour une utilisation selon la revendication 11, dans laquelle lesdits mammifères non humains sont des vaches laitières ou des bovins de reproduction.

13. Composition pour une utilisation selon l'une quelconque des revendications 9 à 12, laquelle composition est administrée audit animal non humain sous la forme d'une seule administration.

14. Composition pour une utilisation selon l'une quelconque des revendications 9 à 13, laquelle composition est administrée auxdits mammifères non humains par injection intramusculaire ou sous-cutanée, de préférence par injection sous-cutanée.

15. Trousse comprenant une composition selon l'une quelconque des revendications 1 à 8 et une notice ou un mode d'emploi comprenant l'information que ladite composition est destinée à être utilisée pour le traitement et/ou la prévention de strongles digestifs et/ou pulmonaires chez un mammifère non humain, de préférence un bovin ou un ovin.
